# EUROPEAN PATENT APPLICATION

(11) **EP 4 279 590 A1**
(43) Date of publication of application: **22.11.2023**
(21) Application number: 22174224.0
(22) Date of filing: 19.05.2022
(51) Int. Cl.: C12N 15/10, C12Q 1/6806, C12Q 1/6853

(54) **METHOD FOR GENERATION OF A NUCLEIC ACID LIBRARY**

(71) Applicant: Miltenyi Biotec B.V. & Co. KG, 51429 Bergisch Gladbach (DE)
(72) Inventor: Wahl, Matthias Bernhard, 51429 Bergisch Gladbach (DE)

(57) **Abstract**

The invention is directed to a method for obtaining a nucleic acid library of a sample comprising polynucleotides comprising the steps:
a. Providing a plurality of modified primer to the polynucleotides, wherein said modified primer is a starting point for a polymerase for nucleic acid amplification
b. Amplification of the polynucleotides using a polymerase
c. Fragmentation of amplified polynucleotides, thereby obtaining a mixture of fragmented and un-fragmented polynucleotides comprising said modified primer
d. Ligation of a plurality of adapter oligonucleotides to the mixture obtained in step c), thereby obtaining a mixture of polynucleotides comprising said adapter oligonucleotides, wherein said adapter oligonucleotides comprise a binding site for an amplification primer
e. Providing an amplification primer to the mixture obtained in step d), wherein said amplification primer is a starting point for a polymerase for nucleic acid amplification
f. initiate a nucleic acid amplification by providing a polymerase
**Characterized in that** the modified primer provided in step a) comprises a functional group which
• capable to prevent the ligation of the adapter oligonucleotides in step d) to polynucleotides comprising said modified primer or
• capable of being removed from the polynucleotides comprising said modified primer after step d)
thereby preventing binding of the amplification primer provided in step f) and a nucleic acid amplification of fragmented polynucleotides comprising the modified primer.

## Description

### Field of invention

The present invention relates to the field of next generation sequencing and nucleic acid library preparation.

### Background

Next Generation Sequencing is an emerging technology extending to all areas of Biomedical Research and Clinical Diagnostics.

Instruments for next generation sequencing have a limited read length (number of molecules downstream of sequencing primers for which the sequence can be accurately determined). In many applications, this read length is insufficient for determining the sequence of the full DNA fragment of interest. Therefore a process called library preparation is essential part of sequencing applications. To that end, the DNA fragment to be sequenced is multiplied and the multiple copies are subsequently shortened by a process called fragmentation. These fragments are sequenced and the obtained sequences are aligned to a reference in order to determine the sequence of the full fragment.

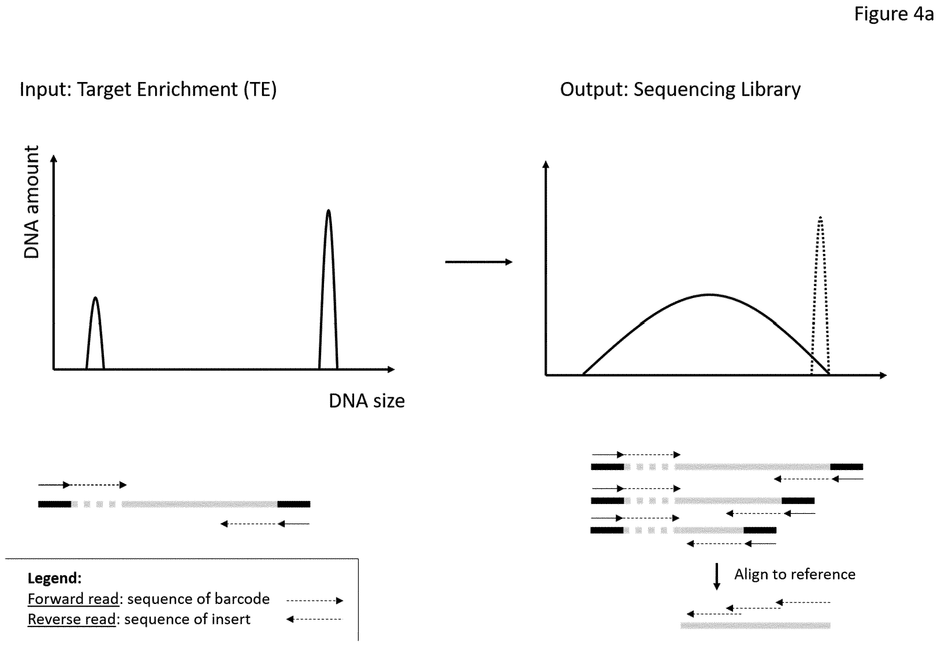

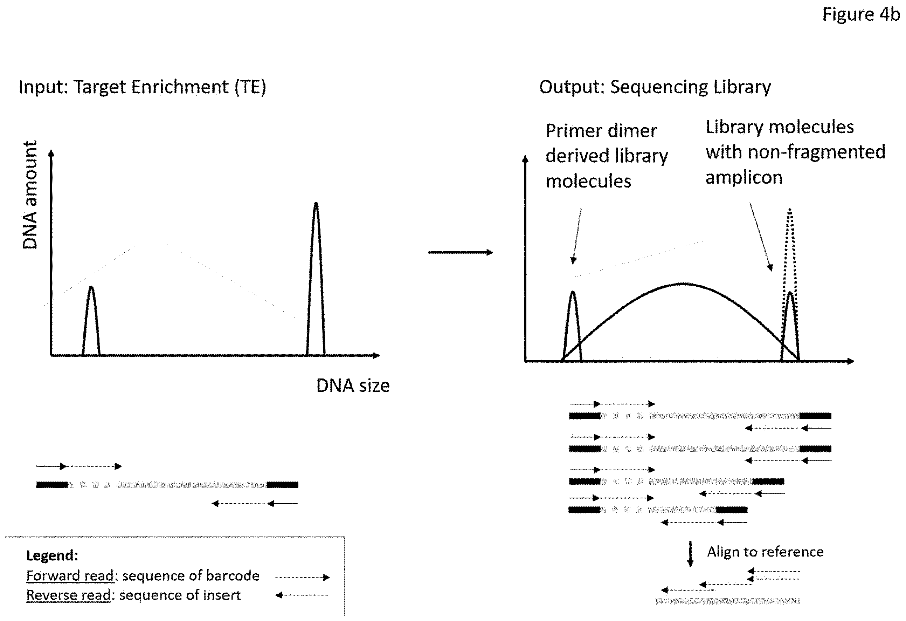

The ideal outcome of a fragmentation-based library preparation workflow is to obtain DNA library molecules that evenly cover the DNA molecule to be sequenced (figure 4a). However, typical libraries of fragmentation-based library prep workflows exhibit a significant fraction of non-fragmented DNA (10x Genomics user guide CG000208 Rev E; Chromium Next GEM Single Cell 5' Reagent Kits v2, section 4.6, page 57), which is a major drawback during library preparation (exemplary shown in figure 4b).

For a library containing many non-fragmented nucleic acids it was shown that many reads obtained by sequencing will be derived from the most 3' end of the enriched target (e.g. a library generated following the 10x Genomics user guide CG000208 Rev E/ the 10x Genomics Chromium Next GEM Single Cell 5' Reagent Kits v2). To ensure that sufficient reads will be obtained from the shorter fragments, the number of sequencing reads has to be increased. This leads to higher costs, because the number of reads for a sample has to be increased and therefore the number of different samples that can be sequenced in parallel has to be decreased. Without compensating by an increased number of reads for each sample, there is high risk of insufficient coverage for assembling the whole sequence of the initial DNA molecule.

Additionally, primer dimers from the initial target enrichment might be present in a library. This is in particular happening in target enrichments in which multiple targets are amplified simultaneously (in these multiplex reactions, it is impossible to design a large number of primers of which none will form primer dimers). These primer dimers often are also carried forward during library prep and will also be sequenced.

The presence of primer dimers (no information about target) and non-fragmented (uneven coverage of target) can only be compensated by increasing the number of reads. This leads to either increased costs or to a lower coverage.

Here we describe an improved or alternative method for nucleic acid library preparation to reduce the number of non-fragmented nucleic acids, in order to avoid sequencing of these molecules. This makes sequencing more accurate and safes cots, because the number of sequencing reads can be reduced. This method may also be used to reduce the number of primer dimers.

### Summary of the invention

The object of the current invention was a method for obtaining a nucleic acid library of a sample comprising polynucleotides comprising the steps
a. Providing a plurality of modified primer to the polynucleotides, wherein said modified primer is a starting point for a polymerase for nucleic acid amplification
b. Amplification of the polynucleotides using a polymerase
c. Fragmentation of amplified polynucleotides, thereby obtaining a mixture of fragmented and un-fragmented polynucleotides comprising said modified primer
d. Ligation of a plurality of adapter oligonucleotides to the mixture obtained in step c), thereby obtaining a mixture of polynucleotides comprising said adapter oligonucleotides, wherein said adapter oligonucleotides comprise a binding site for an amplification primer
e. Providing an amplification primer to the mixture obtained in step d), wherein said amplification primer is a starting point for a polymerase for nucleic acid amplification
f. initiate a nucleic acid amplification by providing a polymerase

Key element is the modified primer provided in step a) which comprises a functional group which is capable to prevent the ligation of the adapter oligonucleotides in step d) to polynucleotides comprising said modified primer or capable of being removed from the polynucleotides comprising said modified primer after step d) thereby preventing binding of the amplification primer provided in step f) and a nucleic acid amplification of fragmented polynucleotides comprising the modified primer.

In addition to that the amplification of primer dimers is prevented.

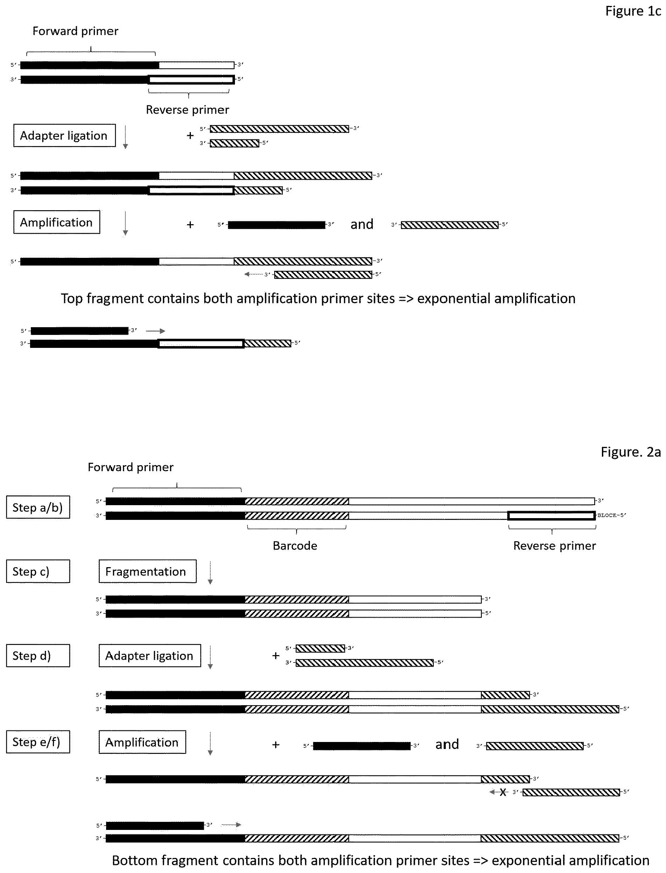

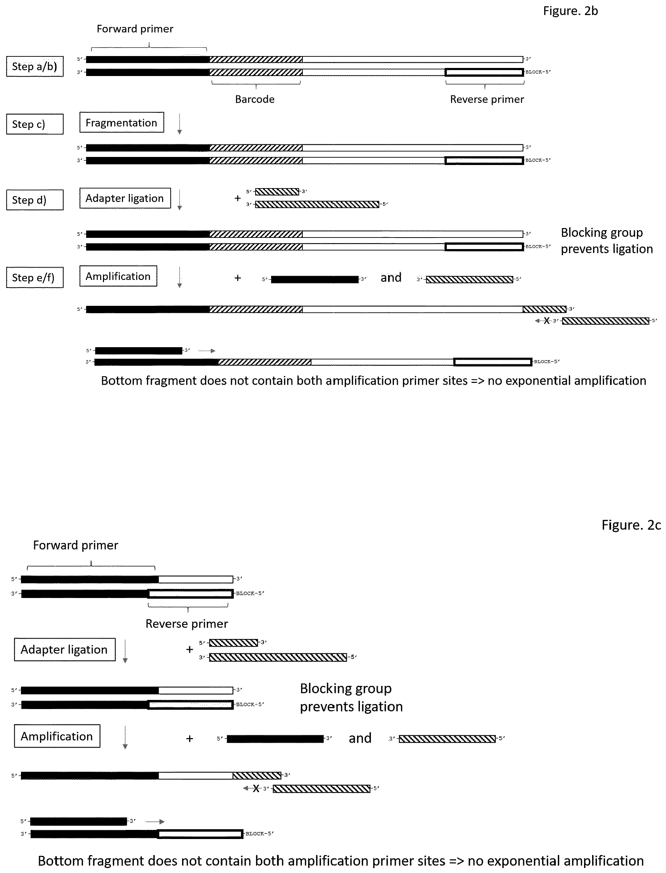

In a first variant of the invention the modified primer provided in step a) comprise a functional group at the 5' end which is a blocking group, thereby preventing the ligation of the adapter oligonucleotides (Figure 2)

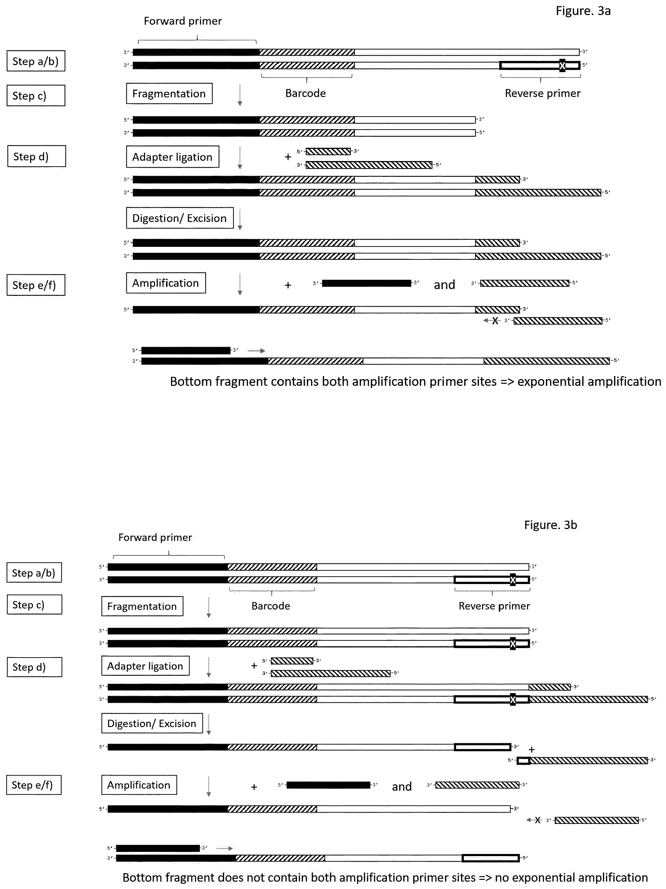

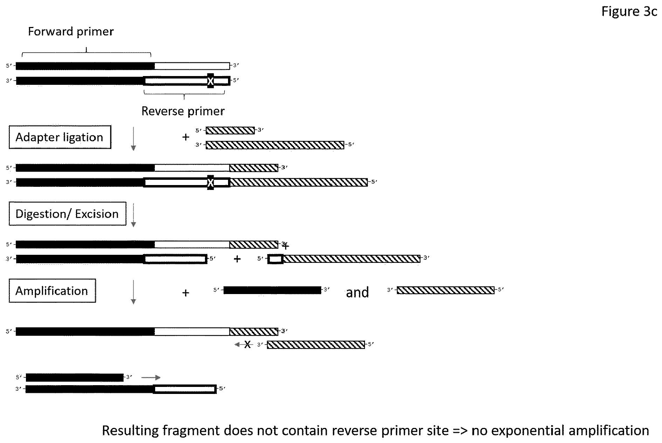

In a second variant of the invention the modified primer provided in step a) comprises at least one nucleotide analogue, which is excised after step d) by an endonuclease, thereby removing the primer binding site provided by the adapter oligonucleotides, and disabling the initiation of the nucleic acid synthesis reaction in step f). (Figure 3).

The invented method may be combined with the statistical fragmentation technique as disclosed in (PCT/EP/2020/081731). In this method one kind of nucleotide analogues is incorporated into the nucleic acid during amplification. The nucleotide analogues are excised by an endonuclease, thus generating a fragmented nucleic acid library.

The target nucleic acid library obtained by the invented method may be used for sequencing. For sequencing any method known in the art may be used.

### Definitions

Unless defined otherwise, technical and scientific terms used herein have the same meaning as commonly understood by one of ordinary skill in the art to which this invention belongs.

As used herein the term "comprising" or "comprises" is used in reference to compositions, methods, and respective component(s) thereof, that are essential to the method or composition, yet open to the inclusion of unspecified elements, whether essential or not.

The words "binding" and "hybridize" and its grammatical equivalents may be used interchangeably. Hybridization of two nucleic acid strands occurs if they are complementary to each other. Hybridization may occur under conditions known in the art.

As used herein, the term "complementary" refers to the capacity for precise pairing between two nucleotides via Watson and crick base pairing. In explanation, if a nucleotide at a given position of a nucleic acid strand is capable of forming hydrogen bonds with a nucleotide of another nucleic acid strand, then the two nucleic acids are considered to be complementary to one another at that position. Complementarity between two single-stranded nucleic acid molecules may be "partial," in which only some of the nucleotides bind, or it may be complete when total complementarity exists between the single- stranded molecules. A "primer" as used herein is a single stranded oligonucleotide made of nucleotides, which is able to bind to complementary nucleic acid sequences. It is understood that all primers described in the current invention may serve as starting point for nucleic acid synthesis / amplification. According to the current invention the modified primer may be used. Such modified primers comprise a functional group and are either capable of preventing the ligation of adapter molecules or are capable of being removed from polynucleotides. A modified primer may also be called "modified target enrichment primer". In addition to that non-modified primer may be used in the disclosed method. These primer do not carry a functional group which is capable to prevent ligation of adapter molecules or capable of being removed from the polynucleotides. Amplification primers as used herein also have the characteristics of non-modified primer.

The terms "nucleic acid synthesis" and nucleic acid amplification as used herein, can be used interchangeably. The process of nucleic acid synthesis is well known in the art. In Brief: For nucleic acid synthesis a template nucleic acid is provided, which may be single stranded or double stranded. In case double stranded nucleic acid are used initially a first step is the denaturation into single nucleic acid strands (complement and reverse complement) using techniques known in the art. No denaturation step is needed for single stranded nucleic acids. In the next step primer are provided that bind to complementary regions of the nucleic acid strands. The 3'end of the primer is then elongated using a polymerase and a complementary strand is generated by filling with complementary nucleotides. As a result a complementary nucleic acid strand is formed. For further amplification denaturation of the double stranded nucleic acid is needed, before another round of nucleic acid synthesis can be initiated. In light of the invention nucleic acid synthesis may be symmetric or asymmetric. During a symmetric nucleic acid nucleic acid synthesis, only one primer is used. Thus one strand is synthesized only. During a symmetric reaction a pair of two primers may be provided (a forward and a reverse primer). One primer that binds to the complement and the other binding the reverse complement nucleic acid strand. Thereby a nucleic acid synthesis can be initiated on both strands. Unless depicted otherwise the wording "primer" as used herein may comprise forward primer, reverse primer or both. The wording "Primer of the same specificity" relates to either all used forward or all used reverse primer, in a specific embodiment.

As used herein the term "adaptor" or "adapter" referred to an oligonucleotide which may be ligated to polynucleotides. They contain a primer binding sequence to facilitate amplification or sequencing of associated nucleic acids. The primer binding sequences of the adapter molecules may be same (identical) or different sequences. Thus, for example, the 5' adapters can comprise identical or different primer binding sequences and the 3 ' adapters can comprise identical or different primer binding sequences. Identical primer binding sequences that may be present in different members of a plurality of nucleic acid molecules can allow amplification of multiple different sequences using a single universal amplification primer that is complementary to the universal/ identical primer binding sequence. The adapter molecules may additionally comprise sequences for one or more sample labels and molecular labels (barcodes). The adapters may be double stranded (symmetric), partially double stranded (asymmetric) or single-stranded. One or more adapter molecule can be located on the 5' or 3' end of a nucleic acid. The adapters at the 5' and 3' ends of the adapter-target-adapters can be the same or different.

The term "polynucleotide" and "nucleic acid" can be used interchangeably and refers to a biopolymer composed of nucleotide monomers covalently bonded in a chain. An amplified nucleic acid may be named as "amplicon". A nucleic acid may be DNA or RNA. It can comprise one or more nucleotide analogs. Some non-limiting examples of analogs include: 8-oxo-7,8-dihydroguanine (8-oxoG), uridine (U), inosine (I), 2,6-diamino-4-hydroxy-5-formamidopyrimidine, 5-hydroxyuracil; 5-hydroxymethyluracil; 5-formyluracil; 3-methyladenine; 7-methylguanine; 1,N6-ethenoadenine; and hypoxanthine and its derivates such as deoxy-8-oxo-7,8-dihydroguanine (d8-oxoG), deoxyuridine (dU), deoxyinosine (dI). These nucleotide analogues may be excised by an endonuclease, especially a structure specific endonuclease.

The term "a plurality" of something as used herein means two or more.

### Description of the drawings

All steps used in the drawings are based on the steps mentioned in claim 1.

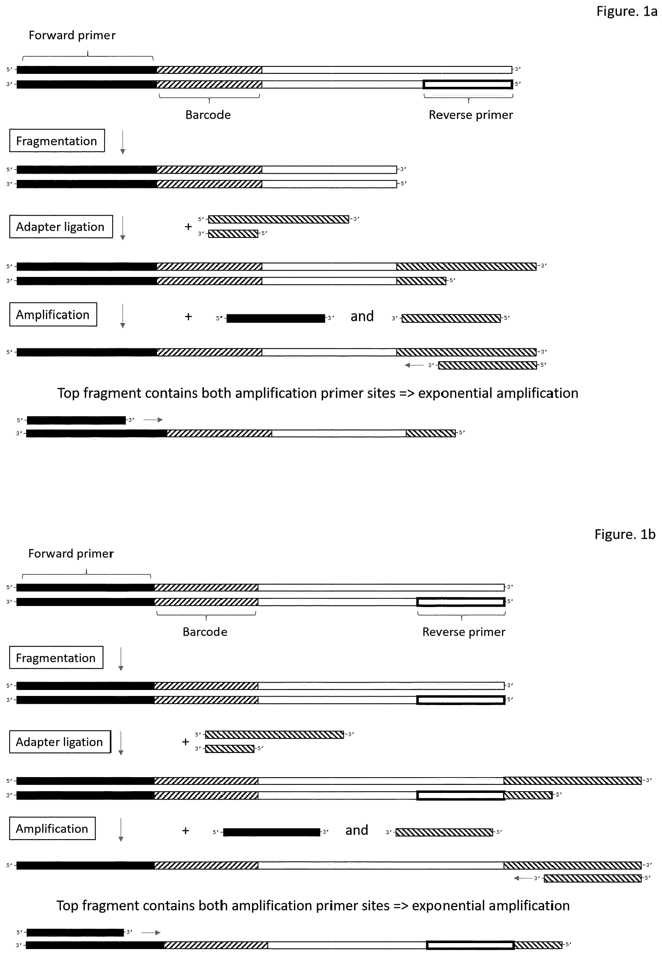

Figure 1: Example of a state in the art library prep workflow. The workflow and its desired outcome is shown in Figure 1a. First, one or multiple molecules are amplified using a forward (black box) and a reverse primer (thick frame). In this example, the molecule or molecules amplified are barcoded (striped box). Multiple molecules are generated in this amplification step; for simplicity, only a single amplicon is depicted in this figure. Next, the amplicons are fragmented . For simplicity, only the 5' fragment is shown. Depending on the method used for fragmentation, the resulting fragment may be blunted using a polymerase exhibiting a 3' to 5' polymerase activity and a 3' to 5' exonuclease activity. Subsequently an adapter molecule containing a primer binding site is ligated using a DNA ligase. To facilitate ligation, a polynucleotide kinase may be added to this or the previous step to phosphorylate the 5' end of the DNA fragments. Additionally, an enzyme with A-tailing activity may be added to this or the previous step in case adapters with T overhangs are being used. Finally, library fragments can be amplified. In this example, one amplification primer binding site is present in the original molecule and the second amplification primer binding is present in the adapter that was ligated . Desired fragmented molecules containing these primers will therefore be amplified.
   With current art library prep workflow, libraries generated from non-fragmented amplicons will amplify in final amplification reaction (Figure 1b). This figure shows the example of an amplicon that was not fragmented. The location of the reverse primer is depicted with a thick frame. The adapter can ligate to such a fragment, and the resulting library molecule will be amplified in the final amplification reaction, therefore, non-fragmented amplicons will be present in the final library.
Figure 1c: With current art library prep workflow, libraries generated from primer dimer molecules will amplify in final amplification reaction. This figure shows the example of a primer dimer that could be generated in the amplification reaction. The location of the reverse primer is depicted with a thick frame. The adapter can ligate to such a fragment, and the resulting library molecule will be amplified in the final amplification reaction, therefore, non-fragmented amplicons will be present in the final library.
Figure 2. Example of library prep workflow using modified primer comprising a blocking group. The same library prep workflow is used in this example as for example 1 except for that a modified reverse primer with a blocking group is used.
Figure 2a shows the effect of modified primer with blocking group on desired outcome of library prep. The presence of the blocking group has no impact on the desired outcome: if an amplicon was fragmented in step **c),** the resulting 5' fragment will not contain the modified primer, therefore the adapter can ligate in step **d).** The resulting library molecules can be amplified in the final amplification reaction (step **f)).** Note that in this example an adapter oligonucleotide (termed MIL-adapter) was used, where the full primer binding site of the adapter is only present in the bottom strand.
Figure 2b: When using modified primers with blocking groups, libraries molecules generated from non-fragmented amplicons will **not** amplify in final amplification reaction (step **f)**). This figure shows the example of an amplicon that was not fragmented in step **d).** The location of the primer is depicted with a thick frame. During adapter ligation, the blocking group prevents ligation of the adapter to the non-fragmented amplicon. As a consequence, the adapter containing the binding site for the amplification primer will not be linked to such an amplicon, therefore no exponential amplification will happen in the final amplification reaction (step **f).**
Figure 2c: When using modified primers with blocking groups, libraries molecules generated from primer dimers will **not** amplify in final amplification reaction (step **f)**)**.** This figure shows the example of a primer dimer that was generated in step **b).** The location of the primer is depicted with a thick frame. During adapter ligation, the blocking group prevents ligation of the adapter to the primer dimer. As a consequence, the adapter containing the binding site for the amplification primer (step **f)**) will not be linked to such a primer dimer, therefore no exponential amplification will happen in the final amplification reaction (step **f)**)**.**
Figure 3. Example of library prep workflow using modified primer containing a nucleotide analogue that can be excised in a subsequent step.
Figure 3a: Effect of modified primer containing a nucleotide analogue on desired outcome of library prep. The presence of the nucleotide analogue has no impact on the desired outcome: if an amplicon was fragmented in step **c),** the resulting 5' fragment will not contain the modified primer, therefore the adapter can ligate in step **d),** and the subsequent treatment with the endonuclease will not have any impact on these fragment. Therefore, the resulting library molecules can be amplified in the final amplification reaction (step **f)**)**.** Note that in this example an adapter oligonucleotide (termed MIL-adapter) was used, where the full primer binding site of the adapter is only present in the bottom strand
Figure 3b: When using modified primers containing a nucleotide analogue, libraries molecules generated from non-fragmented amplicons will **not** amplify in final amplification reaction (step **f)**)**.** This figure shows the example of an amplicon that was not fragmented in step **c).** The location of the primer is depicted with a thick frame, and the nucleotide analogue is depicted with a "X". The adapter will ligate to non-fragmented amplicons containing the amplification primer, but these molecules containing the reverse amplification primer will be cleaved during the subsequent treatment with the endonuclease. Libraries derived from non-fragmented amplicons containing the reverse amplification primer will therefore not be exponentially amplified in step **f).**
Figure 3c: When using modified primers containing a nucleotide analogue, libraries molecules generated from primer dimers will **not** amplify in final amplification reaction (step **f)**)**.** This figure shows the example of a primer dimer that was generated in step **b).** The location of the primer is depicted with a thick frame, and the nucleotide analogue is depicted with a "X". The adapter will ligate to primer dimers, but these molecules containing the reverse amplification primer will be cleaved during the subsequent treatment with the endonuclease. Primer dimer derived libraries containing the reverse amplification primer will therefore not be exponentially amplified in step **f).**
Figure 4 shows Impact of non-fragmented amplicons and primer dimer on NGS Libraries. Figure 4a shows an example of a RNA-Seq application in which a cDNA molecule of interest is amplified using specific primers (target enrichment). In this example, cDNA molecules were previously barcoded at the 5' end before amplification (depicted with dotted grey lines in the bottom of the figure), and the amplification primers were chosen so that the 5' barcode remains in the amplicons after target enrichment. During target enrichment, multiple copies of the same molecule (same 5' barcode) will be generated. The top two graphs depict the size of the DNA fragment before (left) and after (right) library preparation (depicted as electropherogram). The bottom schematics depict how the sequencing primers will bind to the library fragment (black arrows) and which regions of the fragment will be determined by sequencing (dotted arrow; note that the read length of the sequencer is not sufficient to cover the whole fragment). Top left: After target enrichment, a specific fragment is present together with smaller primer dimers. This specific fragment is too large to be sequenced with a single read. Top right: Fragments are statistically shortened (while keeping the 5' barcode) and adapters with sequencing primers are added to the shortened fragments. The insert (depicted with a solid grey line) is determined with the reverse read, and the barcode is determined with the forward read. After sequencing, all reads with the same barcode (forward read) will be aligned to a reference. In case sufficient reads covering the whole sequence (whole length) were obtained, the sequence of the starting molecule can be determined.
Figure 4b shows an example of a current art library prep result for the same RNA-Seq application described in Figure 4a. Current state in the art methods do not lead to complete fragmentation of the target enrichment product. Therefore such a library will contain many non-fragmented polynucleotides. Therefore, reads covering the 3' end of the target will be overrepresented (bottom right). In order to achieve full coverage of the fragment, the number of sequencing reads has to be increased.

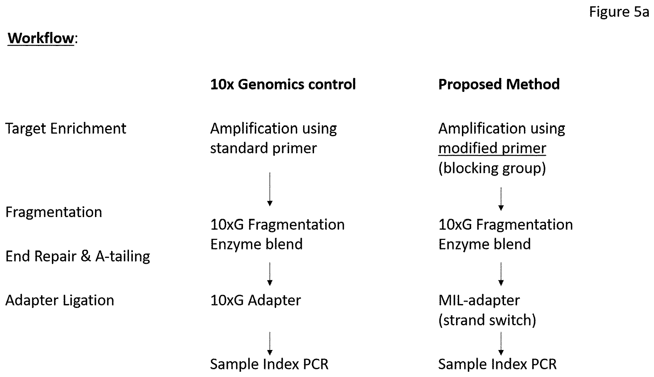

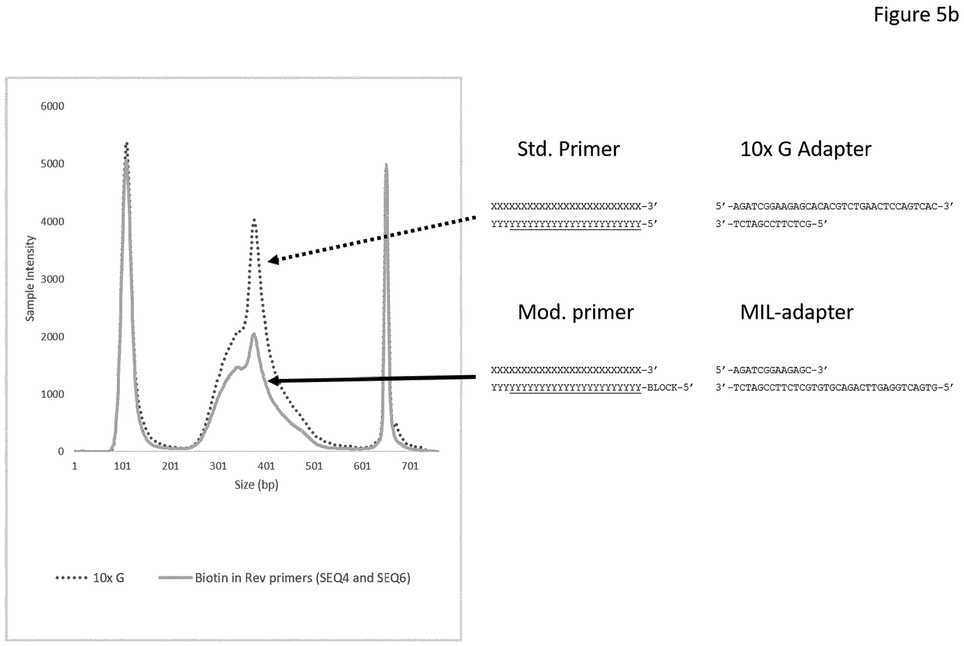

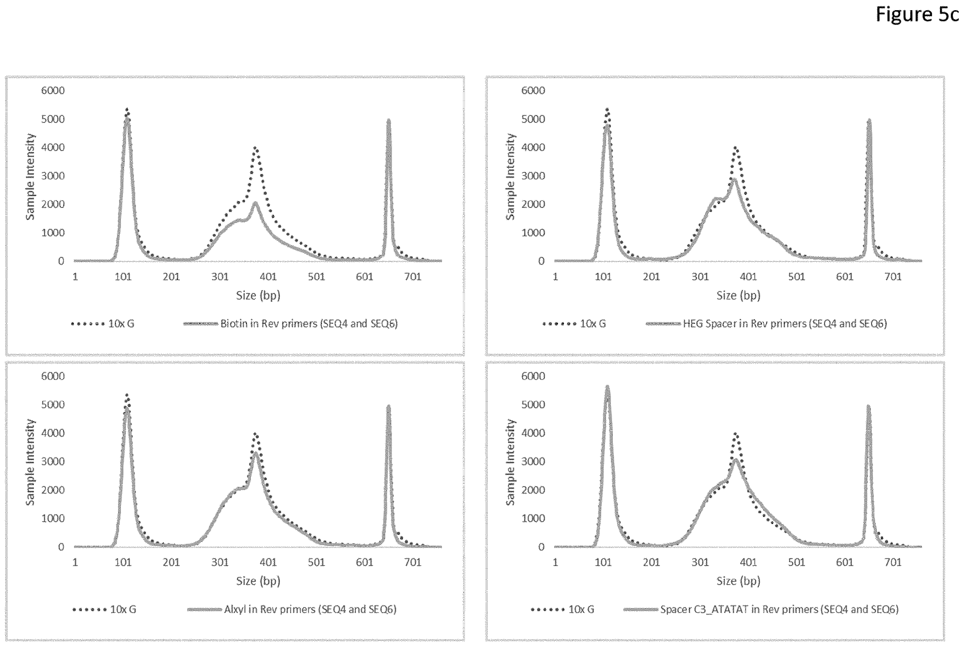

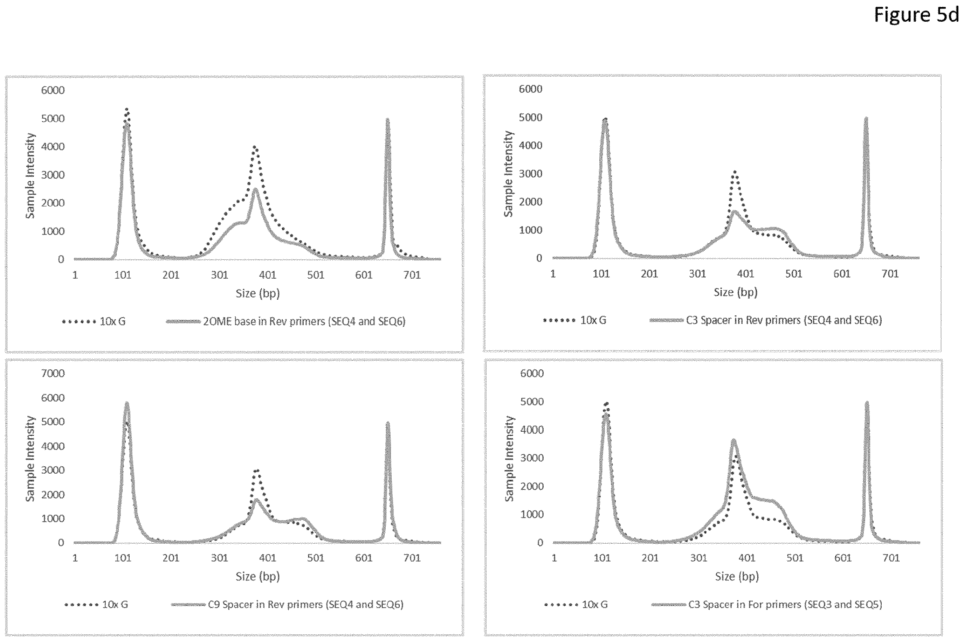

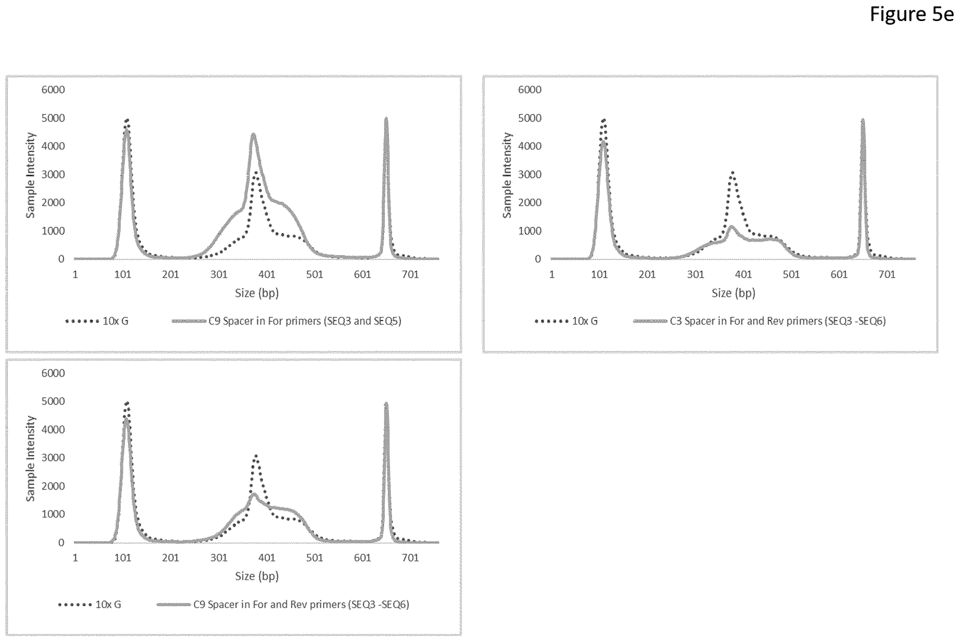

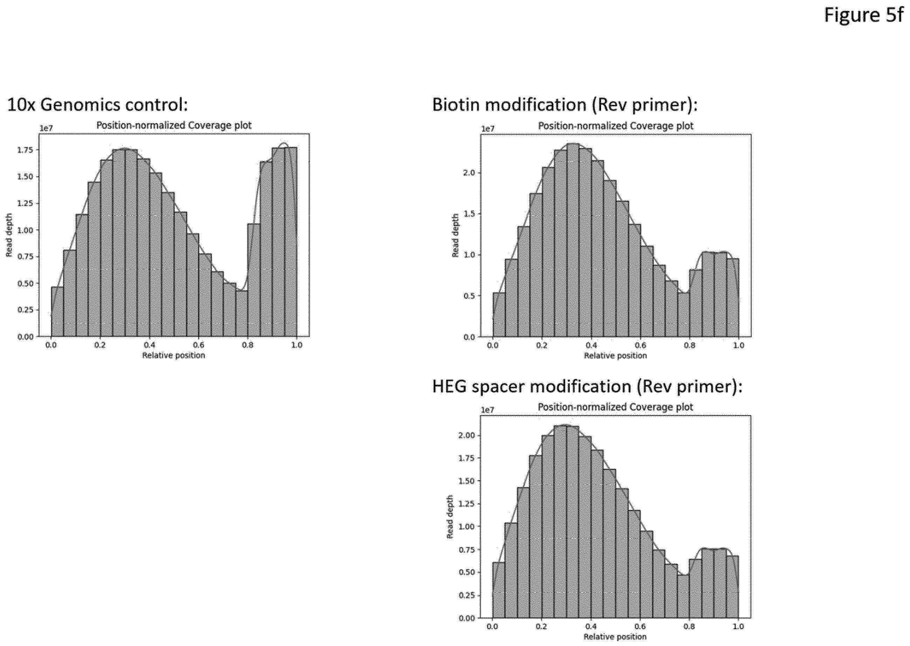

Figure 5 shows a workflow for the evaluation of different blocking groups and unspecific fragmentation as it is described in example 1 of the current invention. Figure 5a shows exemplary shows the workflow, while figures 5b-f show the obtained results.

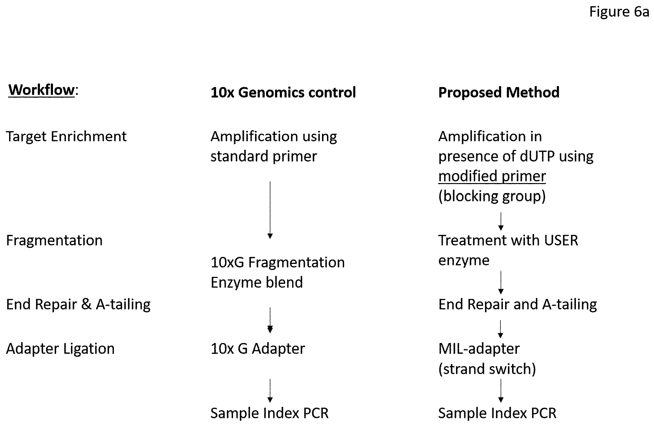

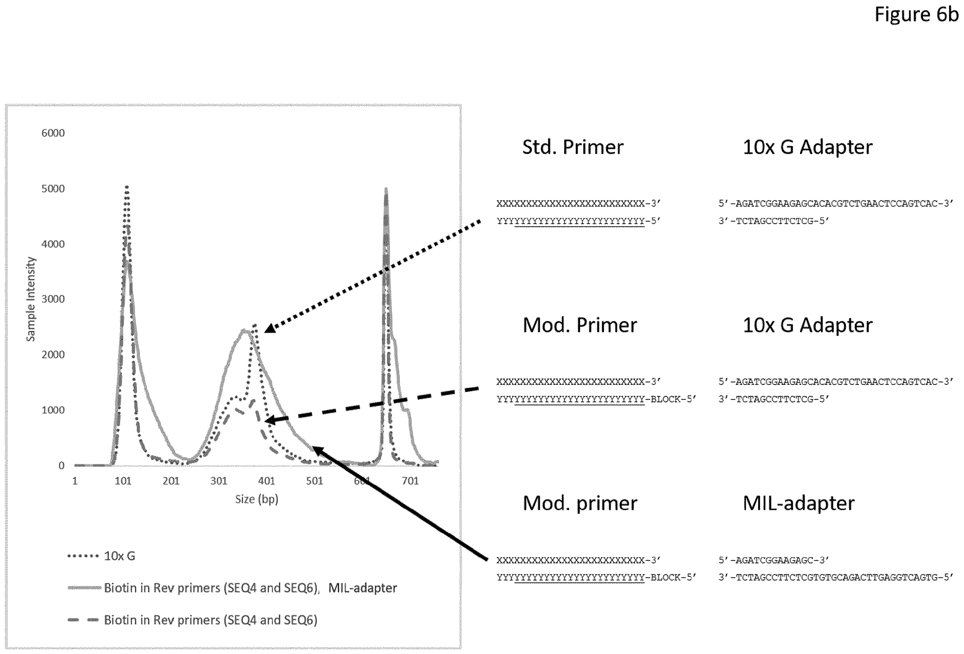

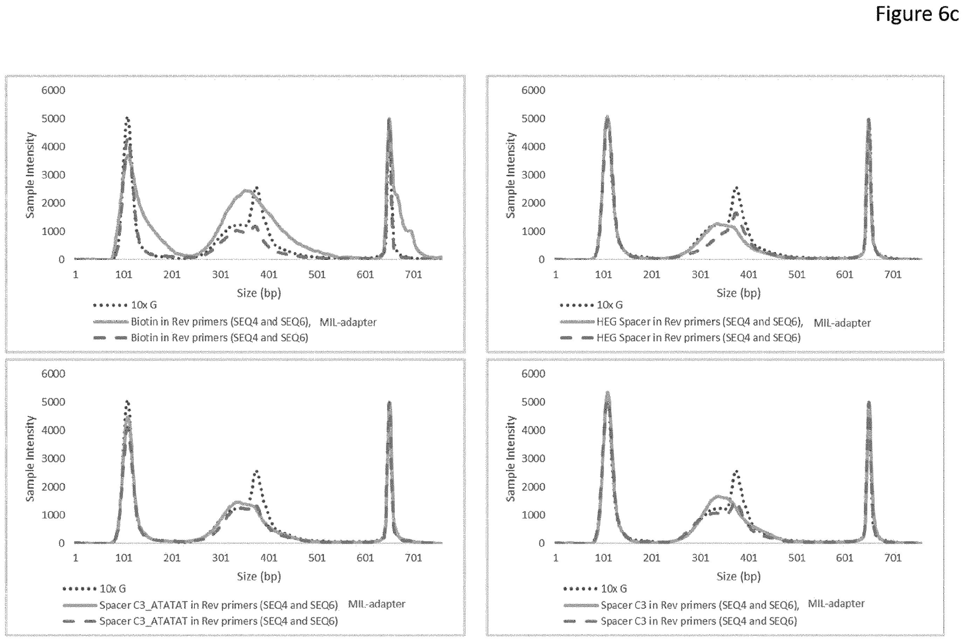

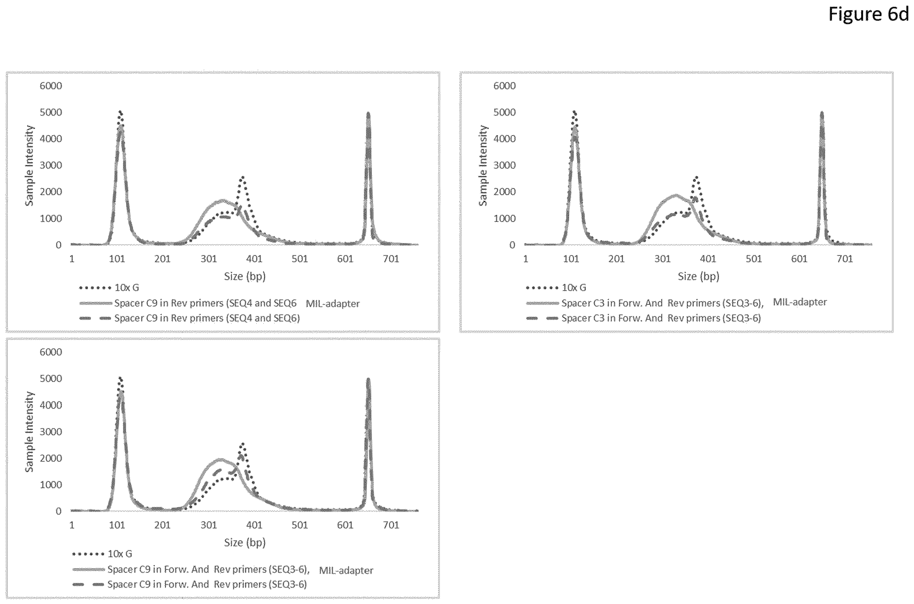

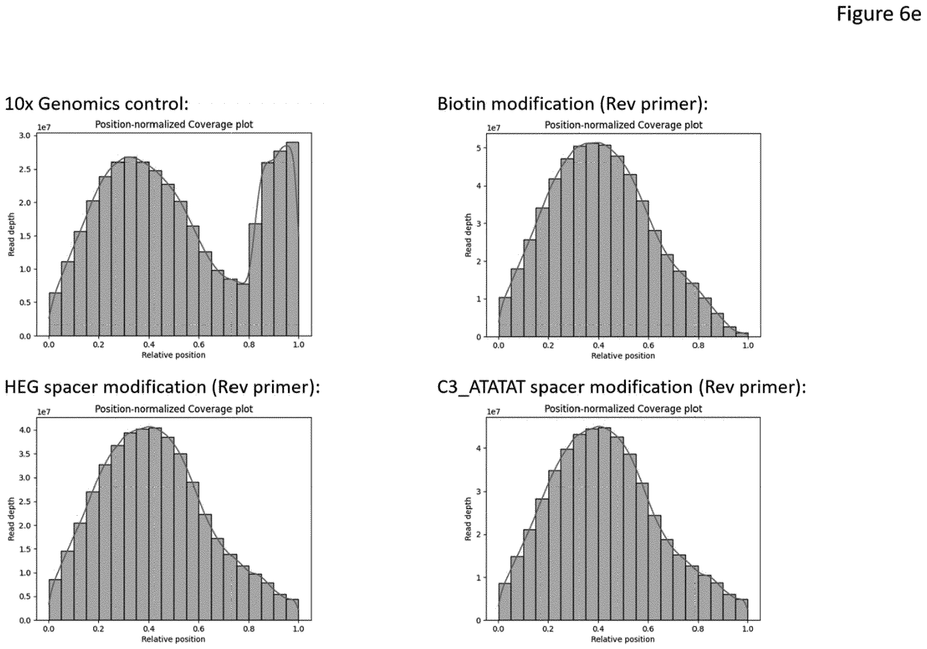

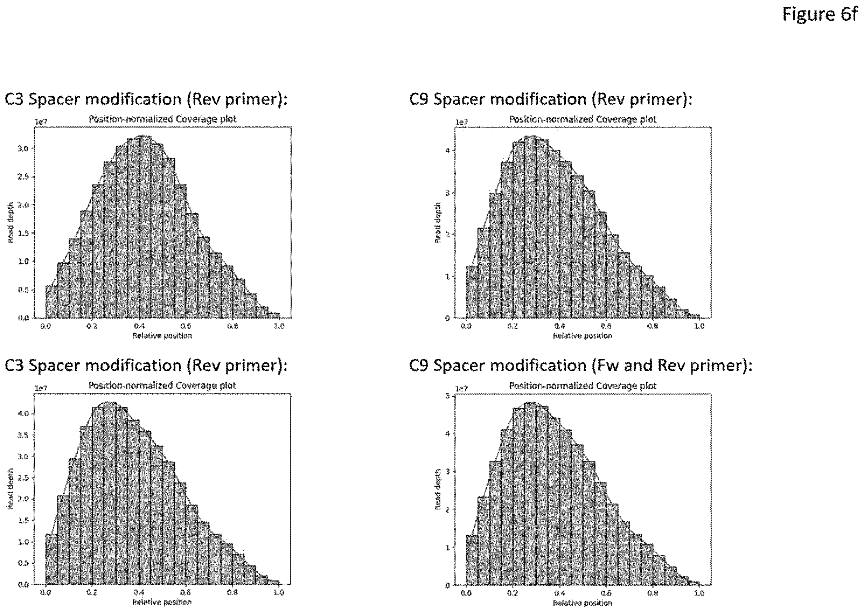

Figure 6 shows a workflow for the evaluation of different blocking groups and Uracil incorporation and excision based fragmentation as it is described in example 2 of the current invention. Figure 6a shows exemplary shows the workflow, while figures 6b-f show the obtained results.

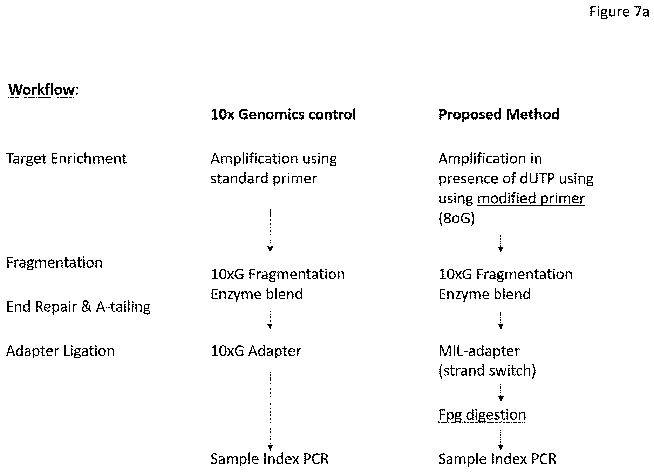

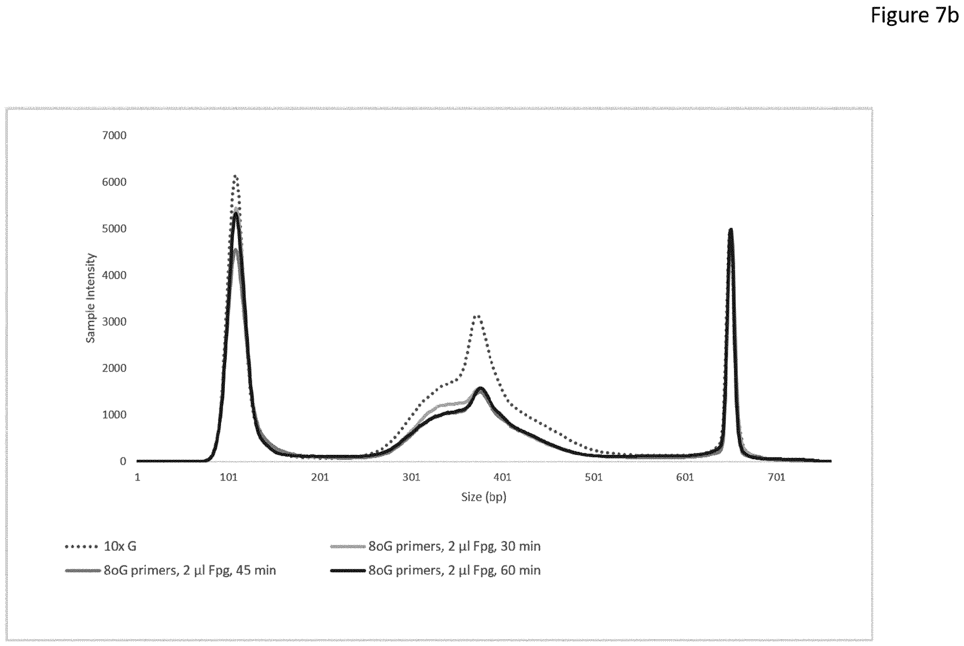

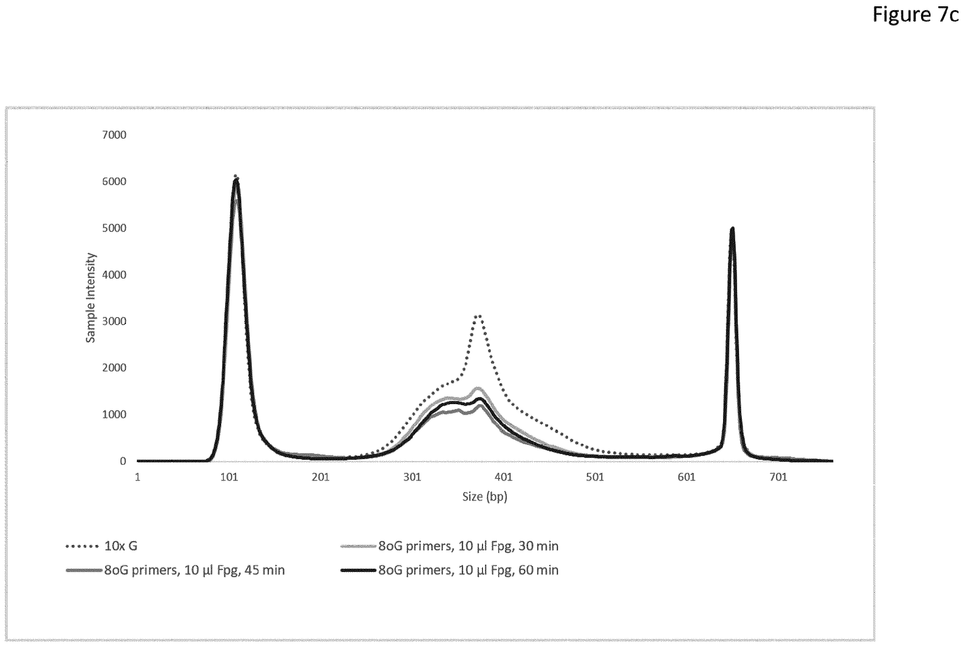

Figure 7 shows a workflow for the evaluation of modified primer comprising a nucleotide analogue and unspecific fragmentation as it is described in example 3 of the current invention. Figure 7a shows exemplary shows the workflow, while figures 7b-c show the obtained results.

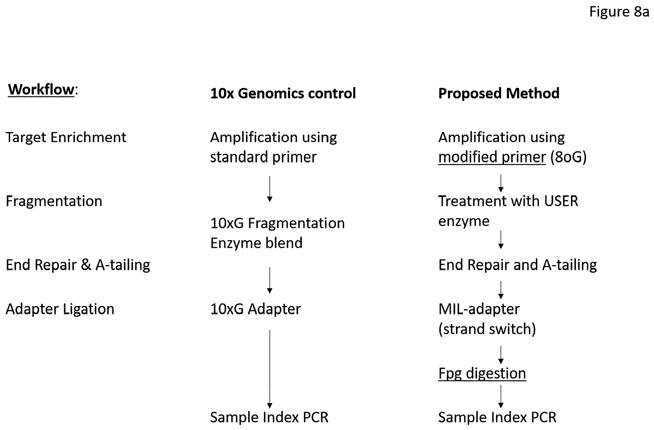

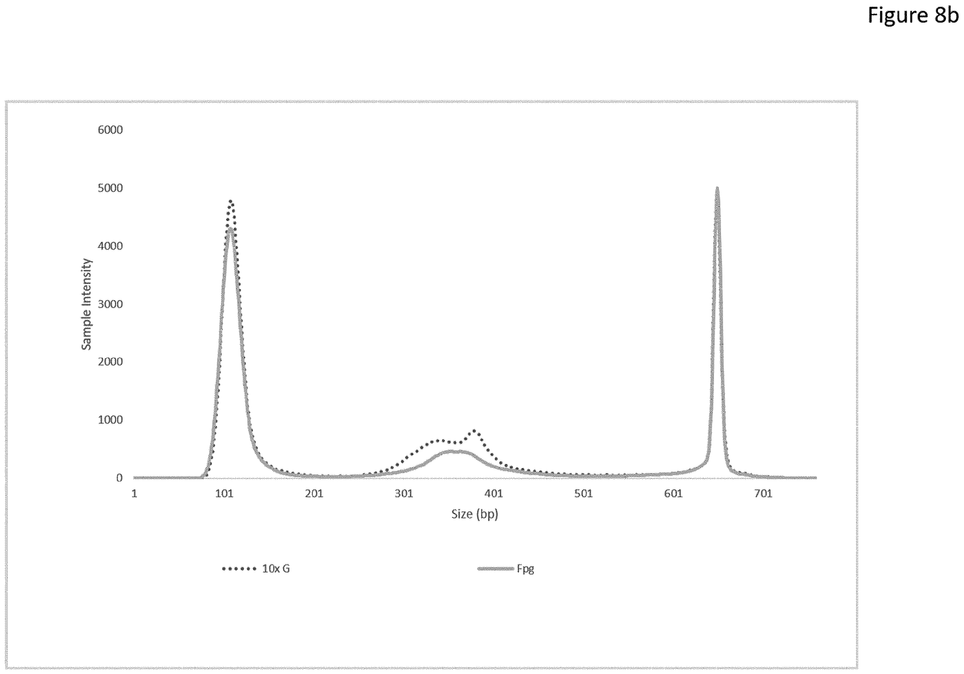

Figure 8 shows a workflow for the evaluation of modified primer comprising a nucleotide analogue and Uracil incorporation and excision based fragmentation as it is described in example 4 of the current invention. Figure 8a shows exemplary shows the workflow, while figure 8b shows the obtained results.

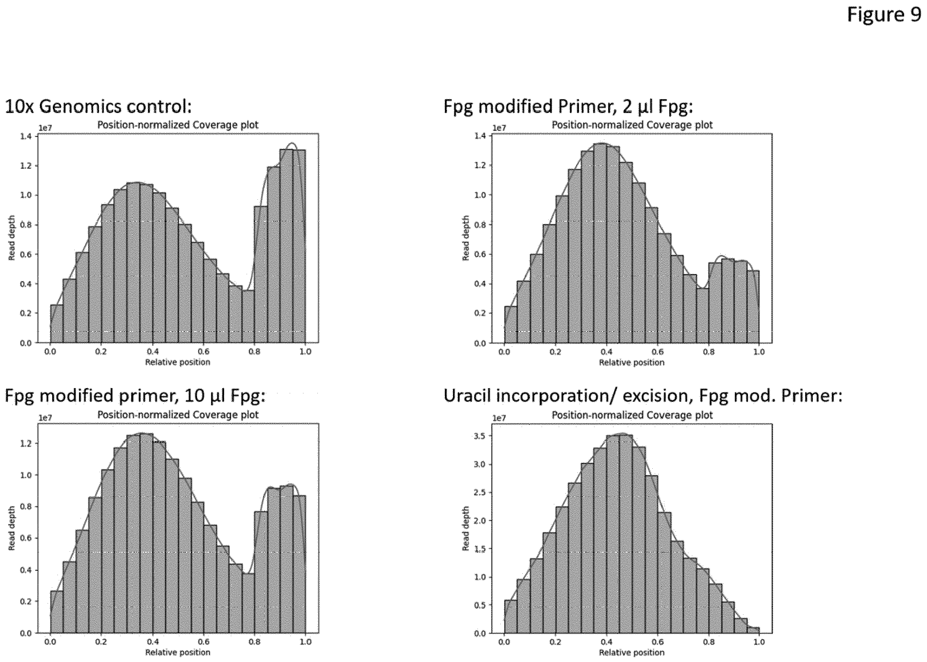

Figure 9 shows additional information on sequence analysis of examples 3 and 4.

### Detailed description

The current invention provides a method to overcome current limitations of nucleic acid library preparation. Main aim is to reduce the number of non-fragmented nucleic acids that can be sequenced. This is achieved by using modified primer for initial nucleic acid amplification during library preparation.

The invented method is for the preparation of a nucleic acid library. This nucleic acid library may be used for several downstream application such as next generation sequencing or polymerase chain reaction.

Samples that may be used for nucleic acid library preparation as described herein, may originate from any specimen, like whole animals, organs, tissues slices, cell aggregates, or single cells of invertebrates, (e.g., Caenorhabditis elegans, Drosophila melanogaster), vertebrates (e.g., Danio rerio, Xenopus laevis) and mammalians (e.g., Mus musculus, Homo sapiens). A biological sample may have the form of a tissues slice, cell aggregate, suspension cells, adherent cells or body fluids.

The nucleic acid used for initial library preparation, may be a polynucleotide strand made of deoxyribonucleic acid (DNA) or ribonucleic acid (RNA), which may have a linear or circular conformation.

In **the first step** (a) of the invented method modified primers are provided, followed by amplification of the nucleic acid using a polymerase (step b). The nucleic acid provided by the sample serves as template for nucleic acid amplification. Techniques and conditions for nucleic acid synthesis and amplification such as polymerase chain reaction are well known in the art.

Standard polymerases that can be used for these reactions are e.g. Taq polymerase, proofreading polymerases like Pfu polymerase or Kod polymerase, fusion polymerases like the KAPA HiFi polymerase (Roche Diagnostics), T7 DNA polymerase, Klenow Fragment, T9 DNA polymerase, Phi29 polymerase. The polymerases may be optimized variants capable of incorporating dUTP nucleotides like the KAPA HiFi Uracil+ polymerase (Roche Diagnostics).

After nucleic acid amplification there follows **step c) fragmentation** of the polynucleotides. During this step the polynucleotides may be double stranded. Several techniques for polynucleotide fragmentation are known in the art such as Physical shearing using the Covaris ultra-sonicator Enzymatical fragmentation and Tagmentation (examples are disclosed in PCT/EP/2020/081731). Fragmentation of polynucleotides yields a mixture of fragmented and non-fragmented polynucleotides. The mixture contains: fragmented polynucleotides comprising the modified primer, fragmented polynucleotides not comprising the modified primer, and non-fragmented polynucleotides comprising said modified primer. It is assumed that the non-fragmented polynucleotides comprise the modified primer.

Resulting fragments may be blunt ended or may have 3' or 5' single stranded overhangs. Before adapter ligation, blunt ends may be generated by the treatment of the fragmented nucleic acids with an enzyme or an enzyme mix exhibiting a 5' → 3' polymerase activity and a 3' → 5' exonuclease activity.

At fragments with 5' protruding ends, a reverse complimentary second strand is being synthesized using the 5' → 3' polymerase activity ("fill-in"); at fragments with 3' protruding ends, the protrusion is removed using the 3' → 5' exonuclease activity. After this treatment, all fragments have blunt ends. Typical enzymes used for such a reaction are e.g. the Klenow Fragment of E. coly DNA polymerase, or T4 DNA polymerase. Fill-in reactions typically also contain polynucleotide kinase to add a phosphate group to the 5' end of the treated fragments. Optionally, an enzyme with A-tailing activity may be also present in step c).

After fragmentation of the nucleic acid in step c) there follows in **step d)** the ligation of a plurality of adapter oligonucleotides to the mixture obtained in step c). As a result a mixture of polynucleotides comprising said adapter oligonucleotides is obtained, wherein said adapter oligonucleotides comprise a binding site for an amplification primer. The adapter oligonucleotide used for ligation contains at least one amplification primer binding sequence. It may further contain additional primer sequences suitable for amplification and sequencing. In addition to that the adapter molecule may contain a barcode sequences. By adding specific barcodes, individual molecules can be tagged (e.g. by a oligonucleotide barcode like a unique molecular identifier), thereby multiple independent DNA molecules can be sequenced in parallel.

Said adapter molecules may be double stranded having bunt ends or least partially double stranded comprising a single stranded nucleic acid strand overhang (for example a T overhang for samples where an enzyme with A-tailing activity in step c). An example of an adapter is depicted in figure 1a.

Said adapter molecules may be ligated to either one side of the polynucleotides i.e. adjacent to the site of the forward primer or adjacent to the side of the reverse primer (as exemplary shown in figures 1-3) or to both sides of the polynucleotides contained in the mixture obtained in step c). If adapter molecules are ligated to both sites of the polynucleotides, the adapter molecules for each side may be same or different. The side of ligation depends on the modified primer used. If modified reverse primer are used, adapter ligation at the side of the reverse primer will be prevented in non-fragmented polynucleotides, while adapters will ligate to fragmented polynucleotides. If modified forward primer are used, adapter ligation at the side of the reverse primer will be prevented in non-fragmented polynucleotides, while adapters will ligate to fragmented polynucleotides. If a mixture of modified forward and modified reverse primer is used, adapter ligation will be prevented at the polynucleotides still containing the forward or reverse primer.

The ligation of the adapter molecule is performed by a ligase. Common examples for such ligases are e.g. T4 DNA ligase, Taq ligase or equivalent enzymes. Prior to step d, fragments could also be made single stranded and a single-stranded adaptor could be ligated e.g. Thermostable 5' App DNA/RNA Ligase or equivalent enzymes. As a result polynucleotides comprising said adapter are generated.

After ligation of the adapter molecules there follows an amplification step, in which an amplification primer is provided to the mixture obtained in step d), wherein said amplification primer is a starting point for a polymerase for nucleic acid amplification. To initiate a nucleic acid amplification a polymerase is provided. The amplification primer may also be used to initiate a sequencing reaction.

The next sections list multiple preferred variants and embodiments of first aspect of the invention.

### Modified and non-modified primer

Key element of the current invention is the use of modified primer in step a). The modified primer may either prevent the ligation of the adapter oligonucleotides in step d) or is capable of being removed after step d). In both scenarios, the modification leads to the absence of the adapter oligonucleotide in polynucleotides and fragments comprising the modified primer. As a consequence, amplification primer added in step f) cannot bind to such polynucleotides and amplification is prevented, due to the absence of the primer binding sequence.

The modified primer provided in step a) may be selected from forward and / or reverse primer. Forward and reverse primer may be same or different.

In a specific embodiment a further plurality of non-modified primers may be provided in step a). Said non-modified primers do not comprise a functional group, which leads to the absence of the adapter oligonucleotide in polynucleotides and fragments comprising the non-modified primer. Depending on the stoichiometry between non-modified primer and modified primer provided in step a) of the invented method the amount of potentially non-fragmented polynucleotides that will be amplified in step f) can be adjusted. In yet another embodiment the further plurality of non-modified primers provided in step a) may have the same specificity as the modified primer, e.g. a non-modified forward and modified forward or non-modified reverse and modified reverse primer.

The non-modified primer may be selected from forward and / or reverse primer. The molar ratios of the plurality of modified and non-modified primer may be 99 to 1, 95 to 5 or 90 to 10. In another embodiment the molar ratios of the plurality of modified and non-modified primer with the same specificity may be 99 to 1, 95 to 5 or 90 to 10.

In another embodiment at least 50%, 75%, 90% or 99% of the primer provided in step a) are modified primer. In a preferred embodiment at least 50%, 75%, 90% or 99% of the primer with the same specificity provided in step a) are modified primer

In a preferred embodiment all reverse primer provided in step a) may be non-modified primer, whereas all forward primer may be modified primer or vice versa. In yet another embodiment 90% of the reverse primer and 90% of the forward primer may be modified.

### Modified primer - blocking group

In one variant of the invention the modified primer provided in step a) comprise a functional group at the 5' end which is a blocking group (Figure 2). This blocking group prevents the ligation of the adapter oligonucleotides to the polynucleotides (step d). Ligation cannot occur. The modification of the primer prevents the phosphorylation of the 5' group of the polynucleotide comprising said modified primer, which would be needed for the ligation of the adapter molecule.

The blocking group is selected from blocking groups known in the art. Commonly used blocking groups are e.g. Fluorophores or Quenchers coupled to the 5' end of a nucleotide, 5' Amino Modifier (including C6 Amino and C12 Amino), 5' Biotin (including BisBiotin and Biotin-TEG), 5' Thiol Modifier (including C6 Thiol), Carbon spacers (including Spacer C3, Spacer C6, Spacer C12), oligoethylene glycol spacers (including Spacer9, Spacer12, Spacer18, HEG Spacer), Digoxigenin, Acrydite, C3-Azid, DBCO, DBCO-TEG, Cholesteryl-TEG, PC-Amino-Modifier. In one embodiment the blocking group may be selected from the group of biotin, oligoethylene glycol spacers having 1 to 25 glycol units and Carbon spacers having 3 to 12 carbon atoms. Commonly used oligoethylene glycol spacers include Spacer9, Spacer12, Spacer18 and HEG Spacer. In a preferred embodiment the blocking group may be selected from the group of Spacer C3 and HEG Spacer

In another embodiment of the invented method, additionally step b) is performed by providing the natural nucleotides (N) a, t, g, c and one kind of nucleotide analogues (A). The preferred molar ratio of N and A is between 150:1 and 10:1, more preferentially between 150:1 and 25:1. Based on that fragmentation of the amplified polynucleotide in step c) is performed by excision of the A nucleotides by an endonuclease. Nucleotide analogous become incorporated in place of its natural counterpart into the newly synthetized nucleic acid (step b). The newly synthesized nucleic acid can then be treated (step c) using endonucleases. These enzymes excise the nucleotide analogues and create nicks in the newly synthesized nucleic acid. This then leads to fragmentation. The newly synthesized nucleic acid can then be treated using endonucleases such as listed in Table 1. Examples for such enzyme mixtures capable of excising the nucleotide analogue uracil are uracil-DNA glycosylase (UDG) and endonuclease III or UDG and endonuclease VIII (Melamade et al, 1994; Jiang et al, 1007). Alternatively, commercial enzymes or enzyme mixes like the USER enzyme or the thermoliable USER enzyme from New England Biolabs may be used (Cat. No M5508 and M5507, New England Biolabs, Ipswich, MA, USA).

Fragment length can be controlled by adjusting the ration between N to A as previously disclosed in (PCT/EP/2020/081731).

The nucleotide analogue may be selected from the list of 8-oxo-7,8-dihydroguanine (8-oxoG), uridine (U), inosine (I), 2,6-diamino-4-hydroxy-5-formamidopyrimidine, 5-hydroxyuracil; 5-hydroxymethyluracil; 5-formyluracil; 3-methyladenine; 7-methylguanine; 1,N6-ethenoadenine; and hypoxanthine and its derivates such as deoxy-8-oxo-7,8-dihydroguanine (d8-oxoG), deoxyuridine (dU), deoxyinosine (dI) . In a preferred embodiment the nucleotide analogue is deoxyuridine (dUTP).

### modified primer - nucleotide analogue

In another variant of the invention the modified primer provided in step a) comprises at least one nucleotide analogue (figure 3). This nucleotide analogue is excised after step d) by an endonuclease. By doing so, the primer binding site provided by the adapter oligonucleotides is removed. This disables the initiation of the nucleic acid synthesis reaction of this strand that incorporated the modified primer.

In one embodiment the modified primer provided in step a) comprises a nucleotide analogue. During nucleic acid amplification such primer is incorporated into the newly synthetized nucleic acid. Afterwards there follow the fragmentation and ligation of the adapter molecules. Then the nucleotide analogues are excised by an endonuclease, thereby creating nicks in the double stranded nucleic acid structure. This then leads to the generation of polynucleotides that lack the adapter molecules comprising the primer binding site. Polynucleotides lacking the adapter molecules cannot be amplified due to that lack of adapter and therefore due to the lack of the amplification primer binding site.

The nucleotide analogue may be selected from the list of 8-oxo-7,8-dihydroguanine (8-oxoG), uridine (U), inosine (I), 2,6-diamino-4-hydroxy-5-formamidopyrimidine, 5-hydroxyuracil; 5-hydroxymethyluracil; 5-formyluracil; 3-methyladenine; 7-methylguanine; 1,N6-ethenoadenine; and hypoxanthine and its derivates such as deoxy-8-oxo-7,8-dihydroguanine (d8-oxoG), deoxyuridine (dU), deoxyinosine (dI) In a preferred embodiment the nucleotide analogue is of 8-oxo-7,8-dihydroguanine (8-oxoG). The treatment after step d) is conducted using Fpg (formamidopyrimidine [fapy]-DNA glycosylase).

In another embodiment of the invented method, additionally step b) is performed by providing the natural nucleotides (N) a, t, g, c and one kind of nucleotide analogues (A). The preferred molar ratio of N and A is between 150:1 and 10:1. more preferentially between 150:1 and 25:1. Based on that fragmentation of the amplified polynucleotide in step c) is performed by excision of the A nucleotides by an endonuclease. Nucleotide analogous become incorporated in place of its natural counterpart into the newly synthetized nucleic acid. The nucleotide analogue serving as functional group in the primer provided in step a) and nucleotide analogue provided in step b) are different

The newly synthesized nucleic acid can then be treated using endonucleases such as listed in Table 1. These enzymes excise the nucleotide analogues and create nicks in the newly synthesized nucleic acid. This then leads to fragmentation. Examples for such enzyme mixtures creating nicks at Uracil sites are uracil-DNA glycosylase (UDG) and endonuclease III or UDG and endonuclease VIII (Melamade et al, 1994; Jiang et al, 1007). Alternatively, commercial enzymes or enzyme mixes like the USER enzyme or the thermoliable USER enzyme from New England Biolabs may be used (Cat. No M5508 and M5507, New England Biolabs, Ipswich, MA, USA).

Fragment length can be controlled by adjusting the ration between N to A as previously disclosed in (PCT/EP/2020/081731).

The nucleotide analogue may be selected from the list of 8-oxo-7,8-dihydroguanine (8-oxoG), uridine (U), inosine (I), 2,6-diamino-4-hydroxy-5-formamidopyrimidine, 5-hydroxyuracil; 5-hydroxymethyluracil; 5-formyluracil; 3-methyladenine; 7-methylguanine; 1,N6-ethenoadenine; and hypoxanthine and its derivates such as deoxy-8-oxo-7,8-dihydroguanine (d8-oxoG), deoxyuridine (dU), deoxyinosine (dI). In a preferred embodiment the nucleotide analogue serving as functional group in the primer provided in step a) is 8-oxo-7,8-dihydroguanine and the nucleotide analogue provided in step b) is deoxyuridine.

**Table 1: List of modified bases and enzymes that can be used for the method (option: modified base and subsequent excision of modified base after adapter ligation):**

| Enzyme | Modified nucleotide | Cat. No at New England Biolabs |
|---|---|---|
| Uracil DNA glycosylase (including Afu Uracil-DNA Glycosylase, Uracil-DNA Glycosylase and Antarctic Thermoliable UDG) Antarctic Thermolabile UDG | deoxyuridine (dU) | M0280S |
| Apurinic/apyrimidinic Endonuclease 1 | Apurinic/apyrimidinic site (AP) | M0282S |
| Endonuclease II (including Tma Endonuclease III) | damaged pyrimidines, including thymine glycol and 5,6-dihydroxythymine | M0291S |
| Endonuclease IV (including Tth Endonuclease IV) | Apurinic/apyrimidinic site (AP) | M0294S |
| Endonuclease V | deoxyinosine (dl) | M0305S |
| Endonuclease VIII | damaged pyrimidines, including thymine glycol and uracil glycol | M0299S |
| Formamidopyrimidine DNA Glycosylase (Fpg) | damaged purines, including 2,6-diamino-4-hydroxy-5-formamidopyrimidine (FapyG) and 8-oxo-7,8-dihydroquanine (8oxoG) | M0240S |
| Human Alkyl Adenine DNA Glycosylase (hAAG) | 3-methyladenine, 7-methylguanine, 1,N6-ethenoadenine, hypoxanthine, deoxyinosine and deoxyxanthosine | M0313S |
| Human single-stranded selective monofunctional uracil DNA glycosylase (hSMUG1) | uracil, 5-hydroxyuracil, 5-hydroxymethyluracil, and 5-formyluracil | M0336S |
| RNase HII | ribonucleotide, or string of ribonucleotides | M0288S |

All definitions, characteristics and embodiments, variants defined herein with regard to the first aspect of the invention as disclosed herein also apply mutatis mutandis in the context of the other variants and embodiments of the invention as disclosed herein.

### Examples

The following examples are intended for a more detailed explanation of the invention but without restricting the invention to these examples.

The method was evaluated in a single cell targeted RNA sequencing application (targeted RNA-Seq). Towards this goal, we used cDNA generated from human T-cells using the Chromium Single Cell 5' Library & Gel Bead Kit (PN-1000014, 10x Genomics, Pleasanton, CA, USA) following the instructions given in the Chromium Next GEM Single Cell V(D)J Reagent Kits v1.1 user guide (CG000208 Rev E). Aliquots of the same cDNA were used for the examples below.

To amplify specific targets followed by library preparation (RNA-Seq), we used the target enrichment primers of the single Cell V(D)J Enrichment Kit, Human T Cell (PN-1000005, 10x Genomics, Pleasanton, CA, USA) following the instructions given in the Chromium Next GEM Single Cell V(D)J Reagent Kits v1.1 user guide (CG000208 Rev E, Steps 4 and 5).

For all examples below, we used the Chromium Next GEM Single Cell V(D)J Reagent Kits v1.1 user guide (CG000208 Rev E) as baseline protocol and modified specific steps in this protocol. The exact modifications are described in the respective examples.

In each of the examples, we in parallel generated reference libraries (labelled "10x G" or "10x Genomics") exactly following the protocol (Chromium Next GEM Single Cell V(D)J Reagent Kits v1.1 user guide, CG000208 Rev E).

### Example 1: Evaluation of method using modified primers comprising a blocking group in RNA-Seq experiments

To evaluate the method, we only amplified the alpha chain cDNA of the T-cell receptor.

SEQ ID NO:3 and SEQ ID NO:4 were used for target enrichment 1 (step 4.1 in Chromium Next GEM Single Cell V(D)J Reagent Kits v1.1 user guide, CG000208 Rev E), and SEQ ID NO:5 and SEQ ID NO:6 for target enrichment 2 (step 4.3 in Chromium Next GEM Single Cell V(D)J Reagent Kits v1.1 user guide, CG000208 Rev E).

All primers were either non-modified (labelled as "10x Genomics control" or "10x G") or modified as described in Table 2. For adapter ligation (Step 5.2 in Chromium Next GEM Single Cell V(D)J Reagent Kits v1.1 user guide, CG000208 Rev E), we used either the adapter provided in the 10x Genomics kit ("10x Genomics" adapter), or used an adapter generated by annealing oligonucleotides with the sequences SEQ ID NO:1 and SEQ ID NO:2 ("adapter-Mil").

**Table 2: Evaluation of different blocking groups, unspecific fragmentation**

| **Target Enrichment 1** | | | **Target Enrichment 2** | | **Workflow** | |
|---|---|---|---|---|---|---|
| **primer modifications (5' end) blocking group** | | | | | | |
| **primer 1 (SEQ ID NO:3)** | **primer 2 (SEQ ID NO:4)** | **primer 3 (SEQ ID NO:5)** | | **Primer 4 (SEQ ID NO:6)** | **Fragmentatio n** | **Adapter** |
| none | none | none | | none | 10x Genomics | 10x Genomics |
| none | Biotin | none | | Biotin | 10x Genomics | adapter-Mil |
| none | HEG spacer | none | | HEG spacer | 10x Genomics | adapter-Mil |
| none | Alxyl | none | | Alxyl | 10x Genomics | adapter-Mil |
| none | Spacer C3 w ATATAT | none | | C3 spacer w ATATAT | 10x Genomics | adapter-Mil |
| none | 5' most base w. 2O ME modification | none | | 5' most base w. 2O ME modification | 10x Genomics | adapter-Mil |
| none | Spacer C3 | none | | Spacer C3 | 10x Genomics | adapter-Mil |
| none | Spacer C9 | none | | Spacer C9 | 10x Genomics | adapter-Mil |
| Spacer C3 | none | Spacer C3 | | none | 10x Genomics | adapter-Mil |
| Spacer C9 | none | Spacer C9 | | none | 10x Genomics | adapter-Mil |
| Spacer C3 | Spacer C3 | C3 spacer | | Spacer C3 | 10x Genomics | adapter-Mil |
| Spacer C9 | Spacer C9 | C9spacer | | Spacer C9 | 10x Genomics | adapter-Mil |

The difference between the proposed method and the standard 10x Genomics workflow are depicted in figure 5a. Modifications evaluated in this example are listed in Table 2.

The impact of the proposed method was evaluated after the final Post Sample Index PCR Cleanup using SPRIselect beads (Cat. No. B23317, Beckman Coulter, Brea, CA, USA) (step 5.5 in Chromium Next GEM Single Cell V(D)J Reagent Kits v1.1 user guide, CG000208 Rev E) by assessing the size distribution of the libraries using an Agilent 4200 TapeStation System using D5000 or High Sensitivity D5000 Screen Tapes (Cat. No. 5067-5588 and 5067-5592, Agilent, Santa Clara, CA, USA).

The final libraries for each modified primer set (solid line) was pairwise plotted versus a 10x Genomics control (dotted line) as shown in Figure 5b.

The results are shown in Figures 5c to 5e. (note that the experiment was split into two rounds, therefore two different controls were generated. The pairwise comparison of libraries generated with modified primers is depicted versus the control generated in the same round).

The majority of modified primers did show the expected results: Compared to the 10x Genomics control baseline, the peak of non-fragmented library decreased. Only libraries generated with primers containing a O-ME modified nucleotide did not show a substantial reduction.

We also could not observe an effect of the Spacer C3 and Spacer C9 modifications in the forward primer. This is expected as adapter ligation to non-fragmented amplicons happens at the side with the reverse primer. Modifications in the forward primer will nevertheless still reduce primer dimer derived library molecules and therefore are of interest.

We next sequenced a subset of representative libraries. As expected, we did not see substantial differences in number of reads, valid barcodes and the number of cells with productive contigs for the T cell receptor alpha chain (analyzed using the 10x Genomics Cell Ranger software).

To assess the coverage of each library, we generated consolidated coverage plots. These coverage plots were generated by combining the mapping positions of each read to its reference into a single diagram in which 0 corresponds to the first base and 1 to the last base.

These coverage plots show a high peak of reads aligning to the 3' end of the reference (figure 5f left coverage plot). This peak is derived from non-fragmented amplicons. As expected, this peak is markedly reduced in the two libraries generated with 5' modified target enrichment primers (Biotin and HEG spacer, figure 5f).

### Example 2: Evaluation of method using target modified primers comprising a blocking group in dUTP spike-in/ USER enzyme fragmentation based RNA-Seq experiments

We also evaluated the method using an alternative fragmentation method disclosed in PCT/EP/2020/081731. In brief, this fragmentation method uses statistical incorporation of dUTP nucleotides during the amplification reaction followed by excision of Uracil bases using an Uracil-N-glycosylase/ Endonuclease mixture.

Libraries were generated as shown in Figure 6a: During target enrichment, we statistically incorporated Uracil bases by using the KAPA HiFi HS Uracil Master Mix (KK2801, Roche Diagnostics, Rotkreuz, Switzerland) supplemented with 1 µl of 1 mM dUTP (total reaction volume: 100 µl).

After the second target enrichment, we excised the uracil bases using the USER II enzyme (Cat. No. M5508, New England Biolabs, Ipswich, MA, USA).

Next, the sample was subjected to NEBNext^{®} Ultra^{™} II End Repair/dA-Tailing Module (Cat. No. E7546, New England Biolabs, Ipswich, MA, USA), followed by adapter ligation using the NEBNext^{®} Ultra^{™} II Ligation Module (Cat. No. E7595, New England Biolabs, Ipswich, MA, USA).

The adapter used during adapter ligation was generated by annealing oligonucleotides with sequences specified in SEQ ID NO:1 and SEQ ID NO:2; as control, we used the 10x genomics Adaptor Mix (PN 220026, 10x Genomics, Pleasanton, CA, USA).

The exact combination of oligonucleotide modifications and adapters is specified in Table 3.

Figure 6b shows the results for the different conditions evaluated in the experiment (Agilent Tapestation results after Sample Index PCR and Cleanup). Library generated with standard primers and standard 10x Genomics Adapter (dotted line) show the characteristic peak of non-fragmented amplicon (figure 6b top). Library generated with modified primers and standard 10x Genomics Adapter (dashed line) show a reduced peak of non-fragmented amplicon: although the long fragment is ligated to the top strand not containing the modification, the presence of the modification in the second strand reduces the ligation efficiency, most likely by steric effects (figure 6b middle). Library generated with modified primers and "adapter-Mil". The peak of non-fragmented amplicon can no longer be detected. The modification therefore completely prevents adapter ligation to fragments containing the amplification primers, and therefore, non-fragmented amplicons are no longer subjected to sequencing (figure 6b bottom).

The results for the respective library preparations are shown in Figure 6c to 6 d: For all modifications evaluated (Biotin, HEG Spacer, Spacer C3, Spacer C9), we could successfully show that the peak of non-fragmented library can be removed by the proposed method.

We next sequenced all libraries using modified primers in combination with the Mil-adapter. As expected, we did not see substantial differences in number of reads, valid barcodes and the number of cells with productive contigs for the T cell receptor alpha chain (analyzed using the 10x Genomics Cell Ranger software).

**Table 3: Evaluation of different blocking groups, Uracil incorporation and excision based fragmentation**

| | **Target Enrichment 1** | | | **Target Enrichment 2** | **Workflow** | |
|---|---|---|---|---|---|---|
| | | **primer modifications (5' end)** | | | | |
| | **primer 1 (SEQ ID NO:3)** | **primer 2 (SEQ ID NO:4)** | **primer 3 (SEQ ID NO:5)** | **primer 4 (SEQ ID NO:6)** | **Fragmentation** | **Adapter** |
| 1 | none | none | none | none | 10x Genomics | 10x Genomics |
| 2 | none | Biotin | none | Biotin | dUTP inc./ U exc. | 10x genomics |
| 3 | none | Biotin | none | Biotin | dUTP inc./ U exc. | adapter - Mil |
| 4 | none | HEG Spacer | none | HEG Spacer | dUTP inc./ U exc. | 10x genomics |
| 5 | none | HEG Spacer | none | HEG Spacer | dUTP inc./ U exc. | adapter - Mil |
| 6 | none | Spacer C3+ATATAT | none | Spacer C3+ATATAT | dUTP inc./ U exc. | 10x genomics |
| 7 | none | Spacer C3+ATATAT | none | Spacer C3+ATATAT | dUTP inc./ U exc. | adapter - Mil |
| 8 | none | Spacer C3 | none | Spacer C3 | dUTP inc./ U exc. | 10x genomics |
| 9 | none | Spacer C3 | none | Spacer C3 | dUTP inc./ U exc. | adapter - Mil |
| 10 | none | Spacer C9 | none | Spacer C9 | dUTP inc./ U exc. | 10x genomics |
| 11 | none | SpacerC9 | none | Spacer C9 | dUTP inc./ U exc. | adapter - Mil |
| 12 | Spacer C3 | Spacer C3 | Spacer C3 | Spacer C3 | dUTP inc./ U exc. | 10x genomics |
| 13 | Spacer C3 | Spacer C3 | Spacer C3 | Spacer C3 | dUTP inc./ U exc. | adapter - Mil |
| 14 | Spacer C9 | Spacer C9 | Spacer C9 | Spacer C9 | dUTP inc./ U exc. | 10x genomics |
| 15 | Spacer C9 | Spacer C9 | Spacer C9 | Spacer C9 | dUTP inc./ U exc. | adapter - Mil |

We next generated coverage plots introduced in example 1. As expected, we observed a high peak of reads aligning to the 3' end of the reference (figure 6e left coverage plot) that is derived from non-fragmented amplicons.

Surprisingly, this peak derived from non-fragmented amplicons completely disappeared in libraries generated with 5' modified target enrichment primers (reverse primer). This data strongly indicates that the combination of 5' modified target enrichment primers and an fragmentation by statistical incorporation of uracil nucleotides followed by USER enzyme treatment is capable of completely removing non-fragmented amplicons.

### Example 3: Evaluation of method using modified primers comprising a nucleotide analogue in RNA-Seq experiments

We next evaluated the method utilizing modified nucleotides followed by a excision of these modified nucleotides after adapter ligation.

Samples were processed according to the protocol provided in the Chromium Next GEM Single Cell V(D)J Reagent Kits v1.1 user guide (CG000208 Rev E) with the following modifications (see also figure 7a):

Modified target enrichment primers (reverse) were used (SEQ ID NO:7 and SEQ ID NO:8) For some conditions, the "adapter-Mil" (Example 2; SEQ ID NO:1 and SEQ ID NO:2) was used.

After Adapter ligation, samples with modified primers (8-oxo-G) were treated with the Formamidopyrimidine DNA Glycosylase (Fpg) for the indicated time (Cat. No. M0240, New England Biolabs, Ipswich, MA, USA).

Figure 7b and 7c shows the results for the different conditions evaluated in the experiment (Agilent Tapestation results after Sample Index PCR and Cleanup). For both amounts of Fpg, the peak of library derived from non-fragmented amplicons was markedly reduced. This reduction could already be observed after 15 minutes incubation.

### Example 4: Evaluation of method using modified primers comprising a nucleotide analogue in dUTP spike-in/ USER enzyme fragmentation based RNA-Seq experiments

We also evaluated the method utilizing modified nucleotides followed by a excision of these modified nucleotides after adapter ligation with the alternative fragmentation method utilizing statistical incorporation of uracil bases followed by the excision of these uracil bases as disclosed in PCT/EP/2020/081731.

The protocol was identical to example 2 except for the use of primers containing 8-oxo-G modifications (SEQ ID NO:7 and SEQ ID NO:8) and the subsequent Fpg digestion (example 3); see figure 8a for an overview of the workflow step. Like observed in example 2, this method also is capable of completely removing the peak for non-fragmented amplicons (see figure 8b).

### Sequence analysis of examples 3 and 4

We next sequenced a subset of the libraries from examples 3 and 4 and compared the results to a library generated using the 10x Genomics protocol (figure 9) using the same approach as for examples 1 and 2 (all libraries had comparable numbers of reads, valid barcodes and the number of cells with productive contigs for the T cell receptor alpha chain; analyzed using the 10x Genomics Cell Ranger software).

As expected, we saw a marked reduction of non-fragmented amplicons in the libraries generated using modified nucleotides in the modified target enrichment primers that were treated with Fpg after adapter ligation.

Strikingly, for libraries generated using the 10x genomics fragmentation modules we still observed a small amount of non-fragmented amplicons; libraries generated with the fragmentation approach using dUTP spike-in followed by USER enzyme treatment no longer exhibited reads derived from non-fragmented amplicons.

This observation is of great interest as it will allow to fine-tune the amount of non-fragmented amplicons from zero to any desired amount by mixing non-modified and modified primers.

## Claims

1. A method for obtaining a nucleic acid library of a sample comprising polynucleotides comprising the steps
a. Providing a plurality of modified primer to the polynucleotides, wherein said modified primer is a starting point for a polymerase for nucleic acid amplification
b. Amplification of the polynucleotides using a polymerase
c. Fragmentation of amplified polynucleotides, thereby obtaining a mixture of fragmented and un-fragmented polynucleotides comprising said modified primer
d. Ligation of a plurality of adapter oligonucleotides to the mixture obtained in step c), thereby obtaining a mixture of polynucleotides comprising said adapter oligonucleotides, wherein said adapter oligonucleotides comprise a binding site for an amplification primer
e. Providing an amplification primer to the mixture obtained in step d), wherein said amplification primer is a starting point for a polymerase for nucleic acid amplification
f. initiate a nucleic acid amplification by providing a polymerase **Characterized in that** the modified primer provided in step a) comprises a functional group which
• capable to prevent the ligation of the adapter oligonucleotides in step d) to polynucleotides comprising said modified primer or
• capable of being removed from the polynucleotides comprising said modified primer after step d)
thereby preventing binding of the amplification primer provided in step f) and a nucleic acid amplification of fragmented polynucleotides comprising the modified primer.

2. A method according to claim 1 **characterized in that** in step a) **further providing** a plurality of non-modified primers which do not comprise a functional group.

3. A method according to claim 2 **characterized in that** in step a) a plurality of modified and non-modified primer are provided in a molar ratio of 90 to 10.

4. A method according to claim 2 **characterized in that** at least 50% of the primer provided in step a) are modified primer

5. A method according to any of the claims 1-4 **characterized in that** the plurality of modified primer provided in step a) comprise a functional group at the 5' end which is a blocking group, thereby preventing the ligation of the adapter oligonucleotides

6. A method according to claim 5 **characterized in that** the blocking group is selected from the group of biotin, oligoethyleneglycol having 1 to 25 glycol units and carbon spacers having 3 to 12 carbon atoms

7. A method according to any of the claims 5 and 6 **characterized in that**
Step b) is performed by providing the natural nucleotides (N) a, t, g, c and one kind of nucleotide analogues (A) wherein the molar ratio of N and A is between 150:1 and 10:1 and
step c) is performed by excision of the A nucleotides by an endonuclease

8. A method according to any of the claims 1-4 **characterized in that** the plurality of modified primer provided in step a) comprises-at least one nucleotide analogue, and wherein said nucleotide analogue is excised after step d) by an endonuclease, thereby removing the primer binding site provided by the adapter oligonucleotides, and disabling the initiation of the nucleic acid synthesis reaction

9. A method according to claim 8, **characterized in that** the nucleotide analogue is selected from the group of 8-oxo-7,8-dihydroguanine; deoxyuridine; deoxyinosine; 2,6-diamino-4-hydroxy-5-formamidopyrimidine; 5-hydroxyuracil; 5-hydroxymethyluracil; 5-formyluracil; 3-methyladenine; 7-methylguanine; 1,N6-ethenoadenine; hypoxanthine deoxy-8-oxo-7,8-dihydroguanine, deoxyuridine and deoxyinosine

10. A method according to any of the claims 8 or 9 **characterized in that**
Step b) is performed by providing the natural nucleotides (N) a, t, g, c and one kind of nucleotide analogues (A) wherein the molar ratio of N and A is between 150:1 and 25:1 and
step c) is performed by excision of the A nucleotides by an endonuclease wherein the nucleotide analogue serving as functional group in the primer provided in step a) and nucleotide analogue provided in step b) are different

11. A method according to any of the claims 8-10 **characterized in that** the nucleotide analogue serving as functional group in the primer provided in step a) is 8-oxo-7,8-dihydroguanine and nucleotide analogue provided in step b) is deoxyuridine
